# EUROPEAN PATENT SPECIFICATION

(11) **EP 1 189 880 B1**
(45) Date of publication and mention of the grant of the patent: **04.05.2005**
(21) Application number: 00941495.4
(22) Date of filing: 16.06.2000
(51) Int. Cl.: C07C 311/48, C07C 303/38, C08F 214/22, C08F 214/26, C08F 216/14, C08F 16/30

(54) **FLUORINATED IONIC POLYMERS**
FLUORIERTE IONISCHE POLYMERE
POLYMERES IONIQUES FLUORES

(30) Priority: 16.06.1999 US 139567 P
(43) Date of publication of application: 27.03.2002
(73) Proprietor: E.I. DU PONT DE NEMOURS AND COMPANY, Wilmington, Delaware 19898 (US)
(72) Inventor: YANG, Zhen-Yu, Wilmington, DE 19810 (US)
(74) Representative: Carpmaels & Ransford
(86) International application number: PCT/US2000/016659
(87) International publication number: WO 2000/077057

(56) References cited:
- WO-A-90/15828
- WO-A-97/23448
- WO-A-99/45048
- US-A- 4 349 650
- US-A- 4 522 995
- US-A- 5 463 005

## Description

### FIELD OF THE INVENTION

Described herein are new a new class of partially fluorinated ionomers suitable for use in electrochemical applications, particularly in direct methanol fuel cells and lithium ion batteries.

### TECHNICAL BACKGROUND

Monomers of the formula

CH₂=CH(CF₂)₂ₙOCF₂CF₂SO₂F (I)

where n>1 are disclosedin WO 9831716. n=1-4 compositions are explicitly disclosed in Chen et al, "Perfluoro and polyfluorosulfonic acids", Huaxue Xuebao (1982), 40(10), 904-12.

Monomers of the formula

CH₂ = CH(CF₂)₂ₙOCF₂CF₂SO₃M (II)

where n 1, where M is H are disclosed in WO 9831716 although the alkali metal form is not taught. Furthermore, WO 9831716 despite the claim, does not provide specific teaching about how to achieve the sulfonic acid monomer.

Conversion of sulfonyl fluoride to the sulfonate salt of an alkali metal, or to a sulfonic acid are known reactions. Formation of ionomers and acid copolymers by hydrolysis of the sulfonyl fluoride functionality in copolymers of TFE and fluoro alkoxy sulfonyl fluorides is known in the art. The art teaches exposure of the copolymer to strongly basic conditions.

See for example, Ezzell et al. U.S. 4,940,525, wherein is used 25 wt % NaOH(aq) for 16 hours at 80-90°C; Banerjee et al. U.S. 5,672,438, wherein is used 25 wt % NaOH for 16 hours at 90°C, or, in the alternative, an aqueous solution of 6-20% alkali metal hydroxide and 5-40% polar organic liquid (e.g., DMSO) for 5 minutes at 50-100°C; Ezzell et al. U.S. 4,358,545 wherein is used .05N NaOH for 30 minutes for 50°C; Ezzell et al. U.S. 4,330,654, wherein is used 95% boiling ethanol for 30 minutes followed by addition of equal volume of 30% NaOH (aq) with heating continued for 1 hour; Marshall et al. EP 0345964 A1, wherein is used 32 wt % NaOH (aq) and methanol for16 hours at 70°C, or, in the alternative, an aqueous solution of 11 wt % KOH and 30 wt % DMSO for 1 hour at 90°C; and, Barnes et al. U.S. 5,595,676, wherein is used 20 wt % NaOH (aq) for 17 hours at 90°C.

It is also very well-known to protect/deprotect an olefinic double bond by, e.g., bromination followed by debromination after performing a reaction on another part of the olefin.

DesMarteau, U.S. 5,463,005 (1995), and Xue, Ph.D. thesis, Clemson University, disclose perfluorinated sulfonyl imide salts formed from copolymers of TFE and PSEPVE which is represented by the formula

CF₂=CFOCF(CF₃)CF₂OCF₂CF₂SO₂F.

Xue uses the method of protecting/deprotecting the fluorolefinic bond, performing the imidization while the bond is protected. Xue clearly demonstrates that the imidization cannot be performed without protecting the double bond.

Anderson et al., U.S. 4,522,995, disclose copolymerization of (I) with TFE, claiming compositions incorporating 0.2-10 mol % of monomer (I). However, Anderson does not enable any composition incorporating more than 1 mol % of (I). No hint is provided as to how a higher concentration of (I) may be obtained. In fact, the highest incorporation of (I) is achieved at concentrations of (I) in the reaction of ca. 3 mol % while Anderson states that at higher concentrations the reaction is inhibited.

Watanabe et al., U.S. 5,109,086, discloses copolymers of vinylidene fluoride (VF₂) with monomers of the formula CH₂=CHR_{f} where R_{f} is C1-12 perfluoroalkyl formed by radical polymerization. Terpolymers are also disclosed.

It is further known in the art that homopolymers and copolymers containing VF₂ are subject to attack by strong bases, see W. W. Schmiegel in Die Angewandte Makromolekulare Chemie, 76/77 pp 39ff, 1979.

WO 97/23448 discloses a method for preparing fluoroalkylsulfonyl imides by reacting a fluoroalkylsulfonamide with a fluoroalkylsulfonyl halide or a fluorosulfonyl halide in the presence of a non-nucleophilic base.

US 4,349,650 discloses that the reaction of a polyfluorocarbonyl compound such as a polyfluoroketone or polyfluorocarboxylic acid fluoride with fluoride ion and a polyfluoroallyl chloride, bromide or fluorosulfate produces a polyfluoroallyloxy compound such as CF₂=CFCF₂OCF₂CF₂SO₂F.

WO 90/15828 discloses a thermoplastic fluoropolymer that has improved tensile strength, yield strength, and cut- through resistance at elevated temperature. The fluoropolymer may be an ionomer which comprises at least 40 mole percent perfluorinated repeat units and at least 0.4 mole percent repeat units which contain at least one anion of a salt group.

### SUMMARY OF THE INVENTION

The present invention provides for a polymer comprising monomer units of VF₂ and 1 to 40 mol % of ionic monomer units of the formula 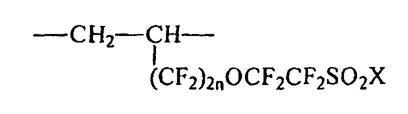 where n≥1, X is O⁻M⁺, or N⁻(M⁺)SO₂R_{f} where M⁺ is H⁺ or an alkali metal cation and R_{f} is C1-4 perfluoroalkyl optionally substituted by one or more ether oxygens.

Further provided is a polymer comprising monomer units of ethylene, tetrafluoroethylene, and 4 to 20 mol % of functionalized monomer units of the formula 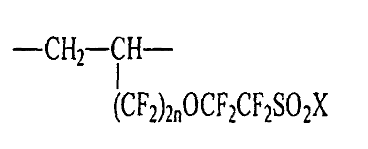 where n≥1, X is F, O⁻M⁺, or N⁻(M⁺)SO₂R_{f} where M⁺ is H⁺ or an alkali metal cation and R_{f} is C1-4 perfluoroalkyl optionally substituted by one or more ether oxygens.

### DETAILED DESCRIPTION OF THE INVENTION

Chemical stability is highly prized in the corrosive environments presented by the electrochemical applications for which the ionomers of the present invention are intended, in particular lithium ion batteries and fuel cells. For this reason, highly fluorinated polymers, well-known for their chemical stability, have long been preferred for use as separators, binders in electrode compositions, both in the form of ionomers and in the form of non-ionic polymers containing electrolyte solutions. However, fluorinated polymers are expensive, the expense correlating roughly with the molar concentration of fluorine in the polymer. Thus, there is incentive to develop polymers, particularly ionomers, which combine high ionic conductivity with good chemical stability at relatively low fluorine concentrations as replacements for the more highly fluorinated polymers in current use in the art. The ionomers of the present invention exhibit just that desired combination.

For the purposes of this invention, "ionic conductivity" refers to the ionic conductivity determined by the method of Doyle et al as disclosed in WO 98/20573.

For the purposes of the discussion herein, it will be understood that a reference to monomer (II) encompasses the embodiment, not disclosed in the art, in which M is an alkali metal.

The present invention provides for a monomer represented by the formula

CH₂ = CH(CF₂)₂ₙOCF₂CF₂SO₂N⁻(M⁺)SO₂R_{f} (III)

where n≥1 and M⁺ = H⁺ or an alkali metal cation, and R_{f} is C1-4 perfluoroalkyl optionally substituted by one or more ether oxygens. Preferably R_{f} is CF₃, and M⁺ is H⁺ or Li⁺.

The monomer (III) imparts a highly desirable combination of high oxidative stability, low fluorine content, and high ionic conductivity when copolymerized with VF₂ or terpolymerized with TFE and ethylene. The copolymer of the Li form of (III) with VF₂ provides particular utility in secondary lithium-ion batteries, while the terpolymer of the acid form of (III) with TFE and ethylene provides particular utility in fuel cells.

As formed, (III) is in the potassium form. The potassium can readily be exchanged with other alkali metals by ion exchange methods well-known in the art. For example, the lithium imide form can be generated by treatment of the potassium imide with a 0.01-2 molar solution of LiCl in dry THF at room temperature. The acid form can be obtained by treating the alkali metal form with aqueous HCl in ether, preferably 10% to 35% HCl, at room temperature.

The process of the invention represents a considerable simplification over the teachings of the art. In a particularly surprising aspect of the present invention monomer (III) can be formed using the known reaction of ―SO₂F moieties with R_{f}SO₂NH₂ in the presence of KF without having to protect the double bond. The subsequent ion exchange chemistry can be conducted without protecting the double bond as well.

Similarly, hydrolysis of (I) to (II) (where M is alkali metal) may proceed without recourse to protecting the double bond.

Polymerization of (I) with VF₂ can be conducted by block polymerization, solution polymerization, suspension polymerization, and emulsion polymerization. Typical peroxide initiators such as Loperso 11 may be used in suspension polymerization or solution polymerization. In an aqueous polymerization, inorganic peroxide such as persulfates (APS and KPS) may be used as an initiator and perfluorocarboxylic salts such as perfluorooctanoic acid may be used as surfactants. Monomers (II) and (III) are preferably polymerized with VF₂ in aqueous polymerization since they are of limited solubility in the fluorinated solvents preferred for polymerization of VF₂ and (I).

The composition of the polymer depends on the ratio of monomers. This was true for all three monomers. One of skill in the art will appreciate that specific reactivity ratios of monomers is determined by the particulars of monomer structure. Accordingly, the present invention provides for an ionomer comprising monomer units of VF₂ and 1 to 40 mol % of monomer units described by the formula 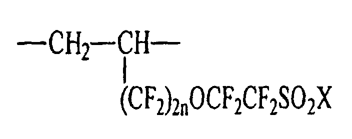 where n≥1, X is O⁻M⁺, or N⁻(M⁺)SO₂R_{f} where M⁺ is H⁺ or an alkali metal cation and R_{f} is C1-4 perfluoroalkyl optionally substituted by one or more ether oxygens. Preferably the concentration of ionic monomer units is 4-20 mol %, most preferably 6-16 mol %. Preferably X is N⁻(M⁺)SO₂R_{f} where M is lithium and R_{f} is CF₃.

In a particularly suprising aspect of the present invention, it is found that monomers (I), (II), and (III), readily react to form terpolymers with TFE and ethylene resulting in polymers incorporating the respective monomer (I), (II) or (III) at concentrations in the range of 4-20 mol % -- much higher than in copolymerization with TFE or ethylene separately. The acid form of the ionomers formed with monomers (II) and (III), or upon hydrolysis and/or imidization of the polymers formed with (I), has been found particularly suitable for use in fuel cells.

Monomer (I) and the polymers formed from monomer (I) according to the methods taught herein, whether in the form of powders, films or other forms, can be hydrolyzed to the alkali metal form of monomer (II) and the polymers formed therefrom as taught herein by contacting monomer (I) or the polymers formed therefrom with a base. In the preferred practice of the invention, the unhydrolyzed material is first converted to the alkali metal salt by contacting with an alkali metal hydroxide or salt solution. Preferably the alkali metal is lithium. If the acid form of the ionic species formed thereby is desired, it is preferable to convert the alkali metal form to the acid form by treatment with acid such as nitric or hydrochloric acid.

The VF₂ copolymers of (I) are unstable in base, and are degraded when the hydrolysis methods of the art, namely contacting with strong bases at elevated temperature, are applied thereto. However, it is found surprisingly that hydrolysis can be successfully effected under far milder conditions than are taught in the art. In particular, hydrolysis has been found to be effected by use of weakly basic reagents have pH less than ca. 12. One method found to be surprisingly satisfactory is contacting the copolymer of VF₂ and (I) with a methanol or methanol/water solution of an alkali metal salt, preferably a carbonate, most preferably lithium carbonate, at a temperature in the range of 0-50°C, preferably room temperature. Lithium carbonate exhibits very low solubility in the solvent, so in the practice of the invention, an excess of the salt is added to the solvent, and additional salt dissolves as the solute is consumed in the hydrolysis. This is one means by which the reaction conditions are kept mild. In a preferred embodiment of the process of the invention, the copolymer of (I) with VF₂ is converted to the hydrolyzed form by treatment with a metal carbonate solution without the need to resort to protecting the double bond.

In a preferred embodiment a copolymer of VF₂ and (I) wherein the concentration of (I) in the polymer is 4-20 mol %, is contacted with a methanol/water solution of Li₂CO₃ at room temperature, followed by a methanol wash.

Other weakly basic solutions suitable for use in the hydrolysis process of the invention include dilute alkali metal hydroxides, and aqueous solutions of alkali metal fluorides. Suitable solvent include alcohols and combinations of polar organic solvents and water such as THF/water, DMF/water, DMSO/water and CH₃CN/water.

The terpolymers of (I) with TFE and ethylene may be hydrolyzed according to methods taught in the art. For example, it is satisfactory to contact the terpolymer with an alkali metal base having a pH greater than 12 in methanol or methanol/water solution at a temperature in the range of 0-100°C, preferably room temperature to 80°C, followed by a methanol wash.

Alternatively, monomer (I) and the polymers formed from monomer (I) according to the methods taught herein, whether in the form of powders, films or other forms, can be converted to the imide form represented by monomer (III) and the polymers of the invention formed therefrom substantially according to the method of Xue, *op.cit*., although surprisingly, according to the process of the present invention, it is not necessary to protect the double bond of monomer (I) when effecting the conversion to the imide form.

In a preferred embodiment of the process of the invention, monomer (I) and the polymers of the invention formed from (I) are contacted at a temperature in the range of 50-180°C, preferably 70-120°C, with a 0.001-5 molar solution of CF₃SO₂NH₂ in an organic solvent in the presence of KF precharged to the reaction vessel to form the potassium imide form of (III) or the polymer formed therefrom. Suitable organic solvents include toluene, chlorobenzene, THF, and oligo ethers. Preferred is acetonitrile. Other ionic forms can be formed by contacting the potassium imide form with an alkali metal salt solution, such as LiCl in methanol, or an acid such as aqueous HCl.

The ionomers of the present invention can be formed either by first forming the desired ionic monomer, the sulfonate or the imide, followed by polymerization with the desired comonomers, namely VF₂ or in the alternative a combination of TFE and ethylene; or, the ionomers may be formed by first forming the desired polymer precursor with monomer (I), followed by hydrolysis or imidization as herein described.

The invention is further described in the following specific embodiments.

### EXAMPLES

### EXAMPLE 1

### Preparation of CH₂=CHCF₂CF₂OCF₂CF₂SO₂F

A mixture of 213 g of ICF₂CF₂OCF₂CF₂SO₂F (Shanghai Institute of Organic Chemistry, China), 0.5 g of (D)-limonene was added to a 1 liter autoclave and pressurized with 30 g of ethylene. The autoclave was heated to 210°C for 8 hrs, after which the autoclave was allowed to cool, and the product removed. The product was distilled to give 187.3 g of ICH₂CH₂CF₂CF₂OCF₂CF₂SO₂F, bp 88-89°C/4 kPa (30 mmHg). 19F NMR: -45.0 (t, J = 5.7 Hz, 1F), -82.7 (m, 2F), -87.2 (m, 2F), -112.7 (m, 2F), -119.3 (t, J = 17 Hz, 2F).

A stirred solution of 136 g of the ICH₂CH₂CF₂CF₂OCF₂CF₂SO₂F so produced in 200 mL of CH₃CN in a 2 liter flask was heated to 75-80°C and held at that temperature for six hours during which 38 g of (C₂H₅)₃N was added via an addition funnel. The reaction mixture was neutralized with concentrated H₂SO₄ and poured into distilled water, and then extracted with diethyl ether. The ether layers were washed with distilled water, and dried over MgSO₄. After removal of the ether, a residue was distilled to give 65.3 g of CH₂=CHCF₂CF₂OCF₂CF₂SO₂F, bp115-117°C. 19F NMR: +45.1 (m, 1F), -82.5 (m, 2F), -87.8 (m, 2F), -112.5 (m, 2F), -118.0 (m, 2F). 1H NMR: 5.80-6.05 (m).

### EXAMPLE 2

### Preparation of CH₂=CHCF₂CF₂OCF₂CF₂SO₃Li

To a stirred suspension of 5.0 g of Li₂CO₃ in 80 mL of MeOH was added 15.0 g of the CH₂=CHCF₂CF₂OCF₂CF₂SO₂F of Example 1, at room temperature. The resulting mixture was stirred at room temperture overnight and filtered to remove solids. The filtrate was evaporated and dried at 100°C in full vacuum to give 12.1 g of white salt, CH₂=CHCF₂CF₂OCF₂CF₂SO₃Li. 19F NMR (acetone-d6): -82.3 (s, 2F), -88.0 (s, 2F), -117.0 (s, 2F), -117.8 (s, 2F).

### EXAMPLE 3

### Preparation of CH₂=CHCF₂CF₂OCF₂CF₂SO₂NMSO₂CF₃

A flask was charged with 5.2 g of dry KF, 6.7 g of CF₃SO₂NH₂ and 40 mL of dry acetonitrile under N₂. 9.8 g of CH₂=CHCF₂CF₂OCF₂CF₂SO₂F was added and the resulting mixture was stirred at 80°C for 15 hrs. 19F NMR analyisis of the reaction mixture revealed no SO₂F group. The reaction mixture was filtered and the solids were washed with acetonitrile. The filtrate was evaporated in vacuo to give 11.3 g of white solid CH₂=CHCF₂CF₂OCF₂CF₂SO₂NKSO₂CF₃. 19F NMR: -789 (s, 3F), -81.2 (s, 2F), -87.9 (s, 2F), -116.9 (s, 2F), -118.0 (s, 2F).

### EXAMPLE 4

### Copolymerization of CH₂=CHCF₂CF₂OCF₂CF₂SO₂F with VF₂ in F113

A 240-mL Shaker tube was charged with 100 mL of 1,1,2-trichlorotrifluoroethane (F113), 10 g of the CH₂=CHCF₂CF₂OCF₂CF₂SO₂F of Example 1, and 1.0 g of Lupersol 11 t-butyl peroxypivalate from Pennwalt Corp. The reaction vessel was cooled in dry ice and degassed by three cycles of evacuation and pressurization with nitrogen gas. 40 g of vinylidene fluoride was added into the vessel and the tube was sealed and heated at 60°C for 8 hours. After completion of the polymerization, the unreacted VF₂ was vented and white solid was washed with MeOH and dried in a partial vacuum oven at 80°C to give 28.8 g of polymer. IR(KBr): 1463 cm⁻¹ (SO₂F). 19F NMR indicated about 7 mol % of CH₂=CHCF₂CF₂OCF₂CF₂SO₂F. DSC showed that the polymer had Tm 149°C and Tg 303°C. By TGA, 10% weight loss was 400°C by TGA in N₂.

### EXAMPLE 5

### Copolymerization of CH₂=CHCF₂CF₂OCF₂CF₂SO₂F with VF₂ in F113

A 75-mL Shaker tube was charged with 30 mL of 1,1,2-trichlorotrifluoroethane (F113), 10 g of the CH₂=CHCF₂CF₂OCF₂CF₂SO₂F of Example 1, and 1.0 g of Lupersol 11. The reaction vessel was cooled in dry ice and degassed and replaced with nitrogen gas repeatedly. 30 g of vinylidene fluoride was added into the vessel and the tube was heated at 60°C for 8 hours. After completion of the polymerization, the unreacted VF₂ was removed and white solid was washed with MeOH and dried in a partial vacuum oven at 80°C to give 16.3 g of polymer. IR(KBr): 1463 cm⁻¹ (SO₂F). 19F NMR indicated about 12 mol % of CH₂=CHCF₂CF₂OCF₂CF₂SO₂F. DSC showed that the polymer had Tm 158°C and 164°C. By TGA, 10% weight loss was 390°C by TGA in N₂.

### EXAMPLE 6

### Copolymerization of CH₂=CHCF₂CF₂OCF₂CF₂SO₂F with VF₂ in water

A 240-mL Shaker tube was charged with 100 mL of deionized water, 10 g of the CH₂=CHCF₂CF₂OCF₂CF₂SO₂F of Example 1, 1.4 g of perfluorooctanoic acid and 0.8 g of potassium persulfate. The reaction vessel was cooled in dry ice and degassed and replaced with nitrogen gas repeatedly. 40 g of vinylidene fluoride was added into the vessel and the tube was heated at 65°C for 5 hours. After completion of the polymerization, the unreacted VF₂ was removed and clear solution was frozen and then defrozen. After being diluted with 600 mL of water, the mixture was heated at 80°C with stirring for 1 hr, filtered, washed with water and dried in over at 80°C in N₂ steam. 16.2 g of white polymer was obtained. IR(KBr): 1463 cm⁻¹ (SO₂F). 19F NMR indicated about 15 mol % of CH₂=CHCF₂CF₂OCF₂CF₂SO₂F. DSC showed that the polymer had Tm 166°C. By TGA, 10% weifgt loss was 400°C by TGA in N₂.

### EXAMPLE 7

### Hydrolysis of copolymer of CH₂=CHCF₂CF₂OCF₂CF₂SO₂F with VF₂

5.0 g of the the polymer powder of Example 4 was added into in a suspension of 0.8 g of Li₂CO₃ in 50 mL of MeOH at room temperature and stirred overnight followed by heating to 60°C and holding at that temperature for 6 hrs. After being diluted with 100 mL of water, the mixture was filtered and washed with water and dried in oven at 70°C with an N₂ purge. A film was formed by pressing at 210°C at 30 kpsi. The film so formed was divided into test specimens of. One specimen was soaked in excess propylene carbonate until solvent uptake reached 50%. Conductivity was determined according to the method of Doyle et al, WO 98/20573, and was found to be 8.1X10⁻⁵ S/cm.

A second specimen was similarly soaked in a 50/50 mixture of ethylene carbonate and gamma-butyrolactonce and the conductivity was determined to be 1.32X10⁻⁴ S/cm.

### EXAMPLE 8

### Copolymerization of CH₂=CHCF₂CF₂OCF₂CF₂SO₂F with TFE and ethylene in F113

A 240-mL stainless steel tube was charged with 100 mL of 1,1,2-trichlorotrifluoroethane (F113), 10 g of CH₂=CHCF₂CF₂OCF₂CF₂SO₂F and 0.8 g of Lupersol 11 and attached to a gas manifold. The tube was cooled in dry ice and the contents degassed by several cycles of evacuation and repressurization with nitrogen gas. After the final evacuation step, the tube was pressurized with 10 g of ethylene and 30 g of TFE. The tube was then sealed and heated to 60°C and held for 8 hours to effect polymerization. After completion of the polymerization, the unreacted ethylene and TFE were removed by venting and the white solid was washed with MeOH and dried in a partial vacuum oven at 80°C to give 47.0 g of polymer. IR(KBr): 1464 cm⁻¹ (SO₂F). Elementary analysis of polymer indicated that polymer composition was 8.67 parts (CF₂CF₂)and 5.36 parts (CH₂CH₂)to 1 part (CH₂CHCF₂CF₂OCF₂CF₂SO₂F) on a molar basis, based on 37.0% of C, 3.12% of H, 52.3% of F and 2.73% of S. DSC showed that the polymer had Tm of 214°C By TGA, 10% weight loss was 430°C by TGA in N₂. A clear transparent and tough film was pressed by placing a sample of the polymer so formed between the platens of a hydraulic press and heated to 250°C with a ram force 9,071.8 kg (20,000 lbs.).

### EXAMPLE 9

### Hydrolysis of Terpolymer of CH₂=CHCF₂CF₂OCF₂CF₂SO₂F with TFE and ethylene

A thin film of the polymer of Example 8 (E90575-41) was immersed in a suspension of 2.1 g of LiOH, 20 mL of water, 20 mL of MeOH and 30 mL of DMSO at 70°C for 6 hrs. The film was removed and washed with water many times and dried overnight in a vacuum oven at 80°C with a N₂ purge. Conductivity was determined as in Example 7. One specimen was soaked in PC to saturation and conductivity was 2.6X10⁻⁵ S/cm. After conversion of Li salt to acid upon treatment with diluted HNO₃, conductivity of the film in water was 13X10-3 S/cm.

### EXAMPLE 10

### Copolymerization of CH₂=CHCF₂CF₂OCF₂CF₂SO₂F with TFE and ethylene in F113

A 240-mL Shaker tube was charged with 100 mL of 1,1,2-trichlorotrifluoroethane (F113), 15 g of CH₂=CHCF₂CF₂OCF₂CF₂SO₂F and 0.5 g of Lupersol 11. The reaction vessel was cooled in dry ice and degassed and replaced with nitrogen gas repeatedly. 7 g ethylene and 22 g of TFE were added into the vessel and the tube was heated at 60°C for 10 hours. After compeletion of the polymerization, the unreacted monomers were removed and white solid was washed with acetone and dried in a partial vacuum oven at 80°C to give 30.3 g of polymer. By TGA, decomposition temperature of the polymer was 390°C and 10% weight loss temperature was 420°C in N₂. DSC showed no melting point and glass transition point at above 25°C. A thin transparent film was pressed at 240°C. Composition was found by elemental analysis to be 2.67 parts (CF₂CF₂) and 4.85 parts (CH₂CH₂) to 1 part (CH₂CHCF₂CF₂OCF₂CF₂SO₂F) on a molar basis.

### EXAMPLE 11

### Hydrolysis of Terpolymer of CH₂=CHCF₂CF₂OCF₂CF₂SO₂F with TFE and ethylene

A thin film of the polymer of Example 10 (E90575-69) was immersed in a suspension of 2.1 g of LiOH, 20 mL of water, 20 mL of MeOH and 30 mL of DMSO at 70°C for 6 hrs. The film was removed and washed with water for many times and dried in oven at 80°C in N₂ stream overnight. Conductivity was 2.05X10⁻⁴ s/cm in PC, 5.41X10⁻⁴ s/cm in EC/GBC. After conversion into acid upon treatment with HNO₃, conductivity in water was 76X10⁻³ s/cm.

### EXAMPLE 12

### Copolymerization of CH₂=CHCF₂CF₂OCF₂CF₂SO₂F with TFE and VF in water

A 240-mL Shaker tube was charged with 120 mL of deionic water, 20 g of the CH₂=CHCF₂CF₂OCF₂CF₂SO₂F of Example 1, 0.5 g of (F(CF₂)ₙCH₂CH₂CH₂NH₃OCOCF₃, n = 4,6) and 0. 1 g of Vazo® V-50 azo-initiator (DuPont Company, Wilmington, DE). The reaction vessel was cooled in dry ice and degassed and replaced with nitrogen gas repeatedly. 40 g of TFE and 40 g of vinyl fluorinde were added into the vessel and the tube was heated at 70°C for 5 hours. Pressure dropped to 4.8 MPa (700 psi) from 19.6 MPa (2850 psi). The polymerization mixture was frozen in dry-ice and thawed at room temperature. Polymer was filtered and washed with water five times, dried in a vacuum oven at 70°C with an N₂ purge. 47.8 g of white polymer was obtained. IR(KBr): 1464 cm⁻¹ (SO₂F). Elementary analysis indicated that composition of the polymer was 8 parts (CF₂CF₂) and 33.6 parts (CH₂CHF) to one part of (CH₂CHCF₂CF₂OCF₂CF₂SO₂F) on a molar basis, based on 40.1% of C, 3.82% of H, 53.0% of F and 1.2% of S. DSC showed that the polymer had Tm 189°C By TGA, decomposition temperature was 260°C and 10% weight loss was 350°C by TGA in N₂. A clear transparent and tough film can be pressed at 210°C at 206.8 MPa (30 Kpsi).

## Claims

1. A polymer comprising monomer units of VF₂ and 1 to 40 mol % of ionic monomer units described by the formula 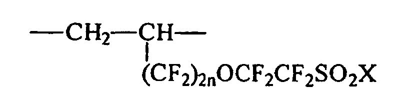 where n≥1, X is O⁻M⁺ or N⁻(M⁺)SO₂R_{f} where M⁺ is H⁺ or an alkali metal cation, and R_{f} is C₁₋₄ perfluoroalkyl optionally substituted by one or more ether oxygens.

2. The polymer of Claim 1 wherein the concentration of said ionic monomer units is 6 to 16 mol %.

3. The polymer of Claim 1 wherein n≥1, X is N⁻(M⁺)SO₂R_{f} where M⁺ is H⁺ or an alkali metal cation, and R_{f} is C₁₋₄ perfluoroalkyl optionally substituted by one or more ether oxygens.

4. The polymer of Claim 1 or 3 wherein M⁺ is H⁺ or Li⁺.

5. The polymer of Claim 3 wherein R_{f} is CF₃, and n=1.

6. A polymer comprising monomer units of ethylene, tetrafluoroethylene, and 4 to 20 mol % of functionalized monomer units represented by the formula 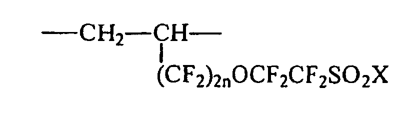 where n≥1, X is F, O⁻M⁺ or N⁻(M⁺)SO₂R_{f} where M⁺ is H⁺ or an alkali metal cation, and R_{f} is C₁₋₄ perfluoroalkyl optionally substituted by one or more ether oxygens.

7. The polymer of Claim 6 wherein X is N⁻(M⁺)SO₂R_{f} where M⁺ is H⁺ or an alkali metal cation, and R_{f} is C₁₋₄ perfluoroalkyl optionally substituted by one or more ether oxygens.

8. The polymer of Claim 6 or 7 wherein M⁺ is H⁺ or Li⁺.

9. The polymer of Claim 6 or 7 wherein R_{f} is CF₃ and n=1.

## Patentansprüche

1. Polymer, das Monomereinheiten aus VF₂ und von 1 bis 40 Mol-% an ionischen Monomereinheiten enthält, welche durch die nachfolgende Formel beschrieben sind: 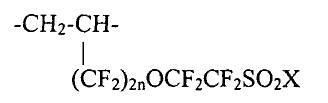 in welcher n ≥1 ist, X ist O⁻M⁺ oder N⁻(M⁺)SO₂R_{f}, wobei M⁺ steht für H⁺ oder für ein Alkalimetallkation, und R_{f} ist ein C₁₋₄ Perfluoralkyl, wahlweise substituiert durch ein oder durch mehrere Ethersauerstoffe.

2. Polymer gemäß Anspruch 1, bei welchem die Konzentration an jenen ionischen Monomereinheiten bei 6 bis 16 Mol-% liegt.

3. Polymer gemäß Anspruch 1, bei welchem n ≥1 ist, X ist N⁻(M⁺)SO₂R_{f}, wobei M⁺ steht für H⁺ oder für ein Alkalimetallkation, und R_{f} ist ein C₁₋₄ Perfluoralkyl, wahlweise substituiert durch ein oder durch mehrere Ethersauerstoffe.

4. Polymer gemäß Anspruch 1 oder 3, bei welchem M⁺ für H⁺ oder Li⁺ steht.

5. Polymer gemäß Anspruch 3, bei welchem R_{f} für CF₃ steht und n = 1.

6. Polymer, welches Monomereinheiten von Ethylen, Tetrafluorethylen und von 4 bis 20 Mol-% an funktionalisierten Monomereinheiten enthält, welche durch die nachfolgende Formel dargestellt sind: 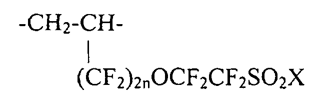 in welcher n ≥1 ist, X ist F, O⁻M⁺ oder N⁻(M⁺)SO₂R_{f}, wobei M⁺ steht für H⁺ oder für ein Alkalimetallkation, und R_{f} ist ein C₁₋₄ Perfluoralkyl, wahlweise substituiert durch ein oder durch mehrere Ethersauerstoffe

7. Polymer gemäß Anspruch 6, bei welchem X gleich N⁻(M⁺)SO₂R_{f} ist, wobei M⁺ steht für H⁺ oder für ein Alkalimetallkation, und R_{f} ist ein C₁₋₄ Perfluoralkyl, wahlweise substituiert durch ein oder durch mehrere Ethersauerstoffe.

8. Polymer gemäß Anspruch 6 oder 7, bei welchem M⁺ für H⁺ oder Li⁺ steht.

9. Polymer gemäß Anspruch 6 oder 7, bei welchem R_{f} für CF₃ steht und n = 1.

## Revendications

1. Polymère comprenant des unités monomères de VF₂ et de 1 à 40% en moles d'unités monomères ioniques décrites par la formule: 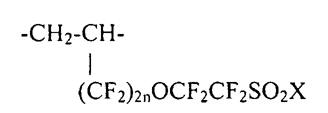 dans laquelle n ≥ 1, X est O⁻M⁺ ou N⁻(M⁺)SO₂R_{f}, où M⁺ est H⁺ ou un cation de métal alcalin, et R_{f} est un groupe perfluoroalkyle C₁₋₄ éventuellement substitué par un ou plusieurs oxygènes d'éther.

2. Polymère suivant la revendication 1, dans lequel la concentration desdites unités monomères ioniques est de 6 à 16% en moles.

3. Polymère suivant la revendication 1, dans lequel n ≥ 1, X est N⁻(M⁺)SO₂R_{f}, où M⁺ est H⁺ ou un cation de métal alcalin, et R_{f} est un groupe perfluoroalkyle C₁₋₄ éventuellement substitué par un ou plusieurs oxygènes d'éther.

4. Polymère suivant la revendication 1 ou 3, dans lequel M⁺ est H⁺ ou Li⁺.

5. Polymère suivant la revendication 3, dans lequel R_{f} est CF₃ et n = 1.

6. Polymère comprenant des unités monomères d'éthylène, de tétrafluoroéthylène et de 4 à 20% en moles d'unités monomères fonctionnalisées représentées par la formule: 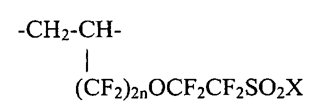 dans laquelle n ≥ 1, X est F, O⁻M⁺ ou N⁻(M⁺)SO₂R_{f}, où M⁺ est H⁺ ou un cation de métal alcalin, et R_{f} est un groupe perfluoroalkyle C₁₋₄ éventuellement substitué par un ou plusieurs oxygènes d'éther.

7. Polymère suivant la revendication 6, dans lequel X est N⁻(M⁺)SO₂R_{f}, où M⁺ est H⁺ ou un cation de métal alcalin, et R_{f} est un groupe perfluoroalkyle C₁₋₄ éventuellement substitué par un ou plusieurs oxygènes d'éther.

8. Polymère suivant la revendication 6 ou 7, dans lequel M⁺ est H⁺ ou Li⁺.

9. Polymère suivant la revendication 6 ou 7, dans lequel R_{f} est CF₃ et n = 1.
